# FASCICULE DE BREVET EUROPEEN

(11) **EP 1 790 382 B1**
(45) Date de publication et mention de la délivrance du brevet: **05.03.2008**
(21) Numéro de dépôt: 06291771.1
(22) Date de dépôt: 16.11.2006
(51) Int. Cl.: A61N 1/37, A61B 5/0205, G06F 19/00

(54) **Dispositif médical implantable actif, notamment dispositif de stimulation, resynchronisation, défibrillation et/ou cardioversion, comprenant des moyens d'aide au diagnostic de l'état du patient**
Aktive implantierbare medizinische Vorrichtung, insbesondere Herzschrittmacher, Resynchronisierer, Defibrillator und/oder Kardiovertierer, mit Hilfsmitteln zur Diagnose des Zustands eines Patienten
Implantable active medical device, in particular device for stimulation, resynchronisation, defibrillation and/or cardioversion, comprising means for helping at diagnosing the state of a patient

(30) Priorité: 23.11.2005 FR 0511848
(43) Date de publication de la demande: 30.05.2007
(73) Titulaire: ELA MEDICAL, 92541 Montrouge (FR)
(72) Inventeur: Casset, Cyrille, 91130 Ris Orangis (FR); Torralba, Jean-Mathieu, 34085 Montpellier (FR)
(74) Mandataire: Dupuis-Latour, Dominique

(56) Documents cités:
- EP-A- 0 750 920
- EP-A- 0 770 407
- EP-A- 0 966 987
- EP-A- 1 317 943
- US-A1- 2001 037 067
- US-B1- 6 273 856

## Description

L'invention concerne les « dispositifs médicaux implantables actifs » tels que définis par la directive 90/385/CEE du 20 juin 1990 du Conseil des communautés européennes, notamment les stimulateurs cardiaques, dispositifs de resynchronisation, cardioverteurs et/ou défibrillateurs destinés au traitement des troubles du rythme cardiaque, ou encore les implants actifs à visée purement diagnostique.

Elle concerne plus précisément ceux de ces dispositifs dont le fonctionnement est asservi à des paramètres recueillis par des capteurs permettant d'évaluer les besoins métaboliques du porteur de l'appareil ainsi que son niveau courant d'activité.

Plus précisément, ces dispositifs comportent deux capteurs de nature différente, à savoir un capteur de mesure d'un paramètre corporel à prépondérance physiologique, et un capteur de mesure d'un paramètre corporel à prépondérance physique. Dans la suite on prendra comme exemple de capteur physiologique un capteur de ventilation-minute (VE), car il s'agit du cas le plus courant, mais ce choix n'a aucun caractère limitatif et d'autres types de capteurs peuvent être également utilisés, dès lors qu'ils produisent un signal représentatif des besoins métaboliques du patient (par exemple un capteur mesurant la saturation d'oxygène dans le sang) ou de son état hémodynamique (par exemple un capteur de bioimpédance intracardiaque). De même, on prendra comme exemple de capteur physique un capteur d'accélération (G), qui est le plus couramment utilisé, mais d'autres types de capteurs sont envisageables pour notamment détecter les mouvements du patient. De façon générale, le capteur physique est caractérisé par un temps de réponse plus court que le capteur physiologique, de manière à permettre une détection très rapide des efforts de courte durée avant même que le paramètre physiologique, de variation plus lente, n'ait évolué de manière significative.

Le EP-A-0 750 920 (ELA Medical) décrit un tel dispositif asservi à deux capteurs, qui opère une sélection de l'un ou l'autre capteur de manière à retenir celui qui donne le signal le plus pertinent à un instant donné. Le EP-A-0 919 255 (ELA Medical) utilise pour l'asservissement une combinaison des signaux délivrés par les deux capteurs.

L'invention concerne plus particulièrement, parmi ces dispositifs, les prothèses dites « multisite » dans lesquelles des électrodes sont placées en une pluralité de sites respectifs distincts comportant les deux sites ventriculaires, droit et gauche, et au moins un site auriculaire. Il peut s'agir d'une prothèse de type « triple chambre » (double stimulation ventriculaire et détection/stimulation auriculaire droite) ou « quadruple chambre » (double stimulation ventriculaire et double détection/stimulation auriculaire).

La plupart des patients recevant un tel dispositif multisite implanté sont des patients qui présentent une conduction auriculo-ventriculaire habituellement normale (chaque évènement auriculaire est suivi d'une dépolarisation ventriculaire associée) et qui n'ont donc pas d'indication standard pour la pose d'un stimulateur implanté. Le dispositif multisite est alors indiqué pour traiter l'insuffisance cardiaque de manière à améliorer l'état hémodynamique général de ces patients par stimulation conjointe et permanente des ventricules gauche et droit afin de resynchroniser ces derniers. Cette thérapie a permis d'observer des résultats souvent spectaculaires pour des patients en insuffisance cardiaque en classe III non améliorée par les traitements classiques.

Le EP-A-1 543 864 (ELA Medical) décrit un tel dispositif multisite.

Le point de départ de l'invention réside dans la constatation du fait que les praticiens ne disposent d'aucun outil qui permette lors des contrôles de routine d'apprécier immédiatement et objectivement si la thérapie appliquée a abouti à l'amélioration attendue de la capacité fonctionnelle à l'effort du patient.

Il existe donc un besoin de disposer d'une fonction, incorporée à ces dispositifs, qui permette d'élaborer et de mémoriser des informations sur l'évolution de l'état clinique du patient entre deux contrôles de routine, c'est-à-dire sur une longue durée, car ces contrôles sont généralement espacés de plusieurs mois.

Une évaluation objective de cette évolution permettra notamment au praticien de s'assurer du bénéfice de la stimulation multisite, de choisir éventuellement une programmation plus adaptée au cas particulier du patient, ou bien d'être par exemple informé de la survenue de troubles de la ventilation importants, non diagnostiqués par le patient lui-même.

Jusqu'à présent, pour évaluer par exemple l'effet d'une programmation particulière sur l'état du patient entre deux visites de routine, le praticien ne pouvait s'appuyer que sur les faits relatés et les impressions du patient - informations empreintes d'une forte subjectivité - ou sur des tests cliniques effectués ou prescrits - informations qui ne reflètent que l'état ponctuel du patient au moment du test, sans vision rétrospective de l'amélioration ou de la dégradation de l'état de celui-ci.

Le besoin existe également de disposer d'une aide au diagnostic synthétique et significative, qui puisse être présentée sous forme d'une symbolique simple et claire au praticien lorsqu'il revoit son patient à l'occasion d'une visite de routine.

L'un des buts de l'invention est donc de proposer un dispositif du type précité connu, c'est-à-dire un dispositif de type multisite pourvu de deux capteurs physiologique et physique, qui puisse en outre analyser et mémoriser les informations issues de ces capteurs entre deux visites distantes de plusieurs mois pour fournir au praticien, le moment venu, une information d'aide au diagnostic représentative de l'évolution de l'état clinique du patient, notamment de sa capacité fonctionnelle à l'effort, depuis la dernière visite.

Le dispositif de l'invention est un dispositif à double capteur tel que décrit dans les documents précités, c'est-à-dire comprenant : des moyens de mesure d'un paramètre corporel à prépondérance physiologique, notamment la ventilation-minute, délivrant un signal physiologique ; des moyens de mesure d'un paramètre corporel à prépondérance physique, notamment l'accélération, délivrant un signal physique ; des moyens discriminateurs des phases d'activité et de repos, opérant en réponse aux signaux physiologique et physique et délivrant un indicateur d'état du patient ; et des moyens d'analyse, pour traiter et combiner les signaux physiologique et physique et l'indicateur d'état, et mémoriser les résultats obtenus sous forme d'un historique de données.

Un tel dispositif est décrit dans le document US-A-2001/037067. Ce dispositif comprend en outre des premiers moyens, pour établir des caractéristiques donnant, pour des dates successives, des valeurs représentatives sur une période donnée du signal physique pendant les phases d'activité du patient, du signal physiologique pendant les phases d'activité du patient, et/ou du signal physiologique pendant les phases de repos du patient.

De façon caractéristique de l'invention, les moyens d'analyse comprennent : des deuxièmes moyens, pour rechercher une pluralité de dates remarquables sur chacune des caractéristiques ainsi obtenues ; des troisièmes moyens, pour attribuer des indices spécifiques d'état clinique respectifs à chacune des périodes comprises entre les dates remarquables sur chacune des caractéristiques ainsi obtenues ; et des quatrièmes moyens, pour combiner en un indice composite les indices spécifiques ainsi déterminés.

Les deuxièmes moyens comprennent avantageusement des moyens de linéarisation multiple, pour transformer la caractéristique en une série continue de segments de droite, notamment de segments dont les extrémités sont des points de la caractéristique, chaque extrémité de segment définissant l'une des dates remarquables pour cette caractéristique.

Ces moyens de linéarisation multiple peuvent être des moyens itératifs opérant par application d'une formule de régression, notamment en définissant une date remarquable si la somme des valeurs absolues des écarts entre segment et caractéristique dépasse un premier seuil prédéterminé, et seulement si chacun des écarts entre segment et caractéristique n'excède pas un deuxième seuil prédéterminé.

Les troisièmes moyens comprennent avantageusement des moyens pour évaluer la pente des segments de droite et attribuer l'indice spécifique correspondant en fonction de la valeur de cette pente.

Les quatrièmes moyens sont avantageusement des moyens mettant en oeuvre une table de vérité préenregistrée donnant de manière univoque, pour chaque combinaison possible d'indices spécifiques, une valeur correspondante d'indice composite.

Les premiers moyens établissent de préférence les caractéristiques à partir de moyennes mobiles des valeurs cumulées du signal physique pendant les phases d'activité, du signal physiologique pendant les phases d'activité, et/ou du signal physiologique pendant les phases de repos.

Les indices spécifiques et composite d'état clinique sont typiquement réévalués à une périodicité quotidienne.

On va maintenant décrire plus en détail un exemple de mise en oeuvre de l'invention, en référence aux dessins annexés.
La figure 1 est un schéma par blocs montrant les différentes fonctions du dispositif impliquées dans la détermination des indices représentatifs de l'évolution des paramètres cliniques du patient.
La figure 2 illustre la manière dont sont prises en compte les variations du signal d'accélération par le dispositif de l'invention.
La figure 3 illustre les variations de la ventilation-minute moyenne en activité, sur une période de plusieurs semaines.
La figure 4 illustre la manière dont est opérée la segmentation d'une caractéristique par linéarisations multiples.
La figure 5 illustre la manière dont sont attribués les valeurs d'indice spécifique à la caractéristique segmentée.
La figure 6 est une table de vérité donnant l'indice composite en fonction des valeurs prises par les divers indices spécifiques.
La figure 7 illustre une représentation graphique des variations de l'indice composite au cours du temps, d'une manière immédiatement appréhendable par le praticien.

On va maintenant décrire un exemple de réalisation de l'invention.

En ce qui concerne ses aspects logiciels, l'invention peut être mise en oeuvre par une programmation appropriée du logiciel de commande d'un stimulateur asservi connu.L'invention peut notamment être appliquée aux dispositifs implantables commercialisés par ELA Médical, Montrouge, France tels que les appareils *Symphony* et *ELA Rhapsody.* Il s'agit de dispositifs à microprocesseur programmables comportant des circuits pour recevoir, mettre en forme et traiter des signaux électriques recueillis par des électrodes et divers capteurs. Il est possible d'y transmettre par télémétrie des logiciels qui seront conservés en mémoire et exécutés pour mettre en oeuvre les fonctions de l'invention qui seront décrites ci-dessous. L'adaptation de ces appareils à la mise en oeuvre des fonctions de l'invention est à la portée de l'homme du métier, et elle ne sera pas décrite en détail.

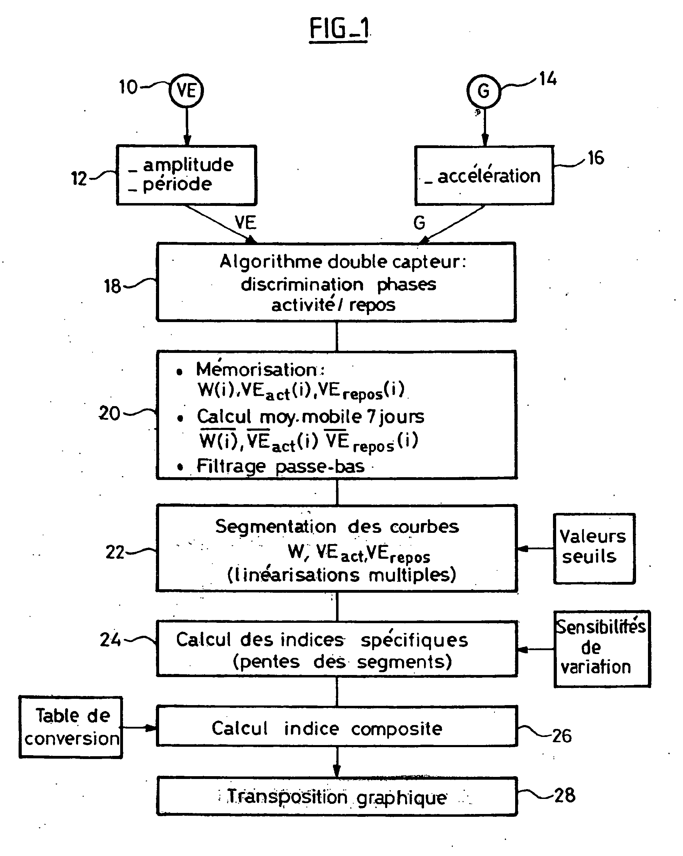

Comme illustré figure 1, le dispositif comporte un capteur 10 délivrant un signal représentatif des besoins métaboliques du patient, typiquement un signal d'impédance transthoracique dont l'analyse des variations périodiques (amplitudes et périodes successives) est opérée par le bloc 12 qui délivre un signal de ventilation-minute (VE). Le dispositif comporte également un capteur physique permettant de détecter les mouvements du patient, typiquement un capteur d'accélération 14 associé à un circuit d'échantillonnage 16 délivrant une succession d'échantillons numérisés avec un pas de 125 ms par exemple.

À partir des informations VE et G délivrées concurremment, le dispositif opère un asservissement de type « double capteur » comme décrit dans les EP-A-0 750 920 et EP-A-0 919 255 antérieurs précités, notamment un asservissement de la fréquence de stimulation et une adaptation éventuelle des paramètres de fonctionnement. Cette fonction d'asservissement proprement dite n'entre toutefois pas dans le cadre de l'invention et ne sera pas décrite en détail.

L'algorithme d'asservissement présente toutefois l'avantage de posséder une fonction de discrimination des phases d'activité et de repos du patient (bloc 18) à partir des indications instantanées par les capteurs MV et G, dont il résulte un indicateur d'état pouvant prendre au moins deux valeurs « activité » et « repos » (d'autres valeurs étant également possibles, par exemple « récupération après effort » qui sera assimilé à une phase d'activité, ou « sommeil » qui n'est qu'un cas particulier de phase de repos).

Pour la mise en oeuvre de l'invention, le dispositif recueille, de façon distincte pour les phases d'activité et pour les phases de repos, les données fournies par les capteurs MV et G et mémorise à partir de ces données :
- la somme W(i) des mesures du capteur G en phase d'activité,
- la ventilation-minute moyenne VEact(i) pendant les phases d'activité, mesurée sur les dernières 24 heures, et
- la ventilation-minute moyenne VErepos(i) pendant les phases de repos, mesurée sur les dernières 24 heures.

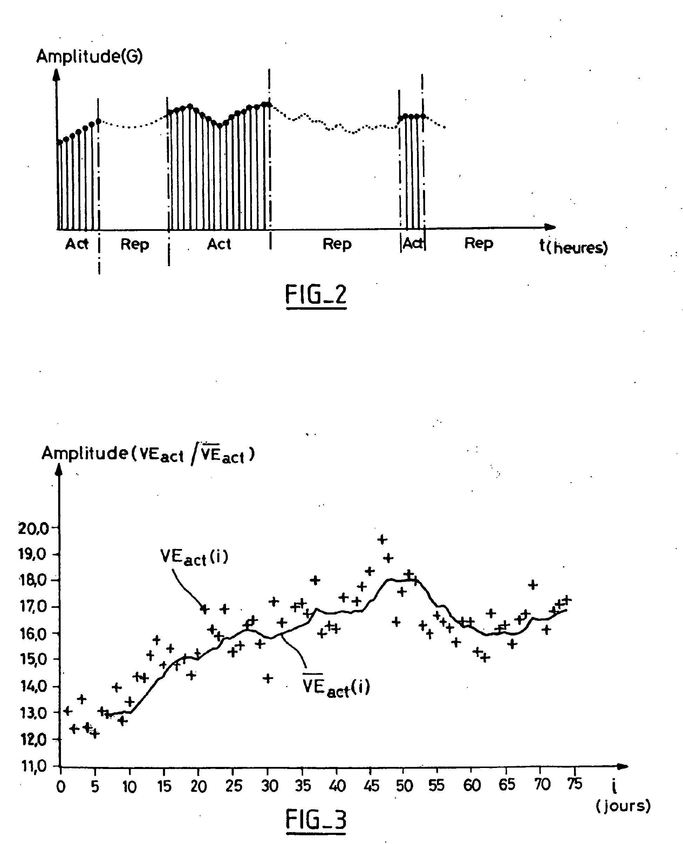

La figure 2 illustre plus précisément la manière dont est obtenue et mise à jour la donnée W(i), représentative de l'effort cumulé développé par le patient au cours des dernières 24 heures : comme on l'a indiqué plus haut, le capteur G foumit une série d'échantillons numérisés par exemple à intervalles de 125 ms, dont la variation au fil du temps est illustrée sur la figure 2. Par ailleurs, l'indicateur d'état permet de distinguer les périodes d'activité (Act) et de repos (Rep). Le dispositif procède à la sommation des valeurs des échantillons depuis les dernières 24 heures, mais en inhibant cette sommation pendant les périodes de repos, en ne sommant donc que des valeurs correspondant à des périodes d'activité.

Le paramètre VEact(i) est une valeur moyenne de la ventilation-minute VE lors des périodes d'activité. Le paramètre VErepos(i) est une valeur moyenne de la ventilation-minute VE lors des périodes de repos. Pour ces deux paramètres, la moyenne obtenue est pondérée en la divisant par la durée passée en activité ou en repos, selon le cas.

De façon typique, ces divers paramètres W(i), VEact(i) et VErepos(i) sont évalués et mémorisés quotidiennement, depuis le premier jour i = 1 suivant l'implantation ou la dernière visite au praticien, jusqu'à une durée pouvant atteindre typiquement i = 90 jours environ, dans le cas de visites de routine trimestrielles.

À partir des valeurs brutes W(i), VEact(i) et VErepos(i) ainsi déterminées et mémorisées, le dispositif calcule ensuite (bloc 20) une moyenne mobile sur sept jours W(i)/, VEact(i)/, VErepos(i)/. Avantageusement, ces moyennes sont en outre soumises à un filtrage passe-bas d'ordre 2 (ou toute autre méthode de lissage de type passe-bas) afin d'en lisser les variations trop abruptes et ne faire apparaître que les évolutions les plus lentes, seules significatives sur le long terme.

Le figure 3 illustre par exemple les variations du paramètre VEact(i) au cours du temps sur une durée de plusieurs semaines. Les croix indiquent les valeurs VEact(i) calculées quotidiennement, le trait continu représentant la moyenne mobile VEact(i)/ sur sept jours, également calculée quotidiennement.

L'étape suivante (bloc 22), caractéristique de l'invention, est une étape de segmentation, qui consiste à modéliser chacune des trois courbes W(i)/, VEact(i)/ et VErepos(i)/ sous forme d'une succession continue de segments de droite dont les extrémités sont des points de la courbe considérée.

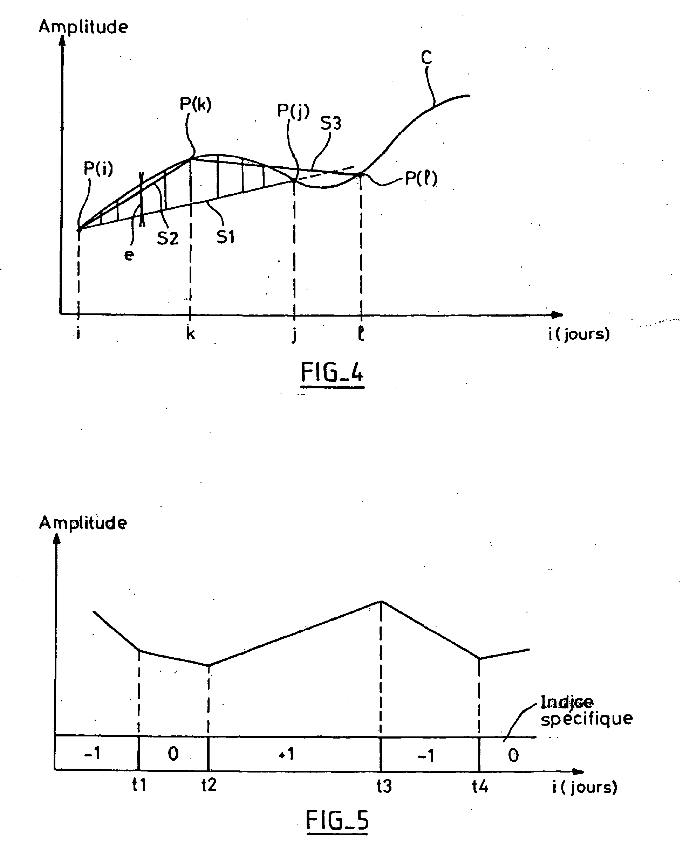

Cette modélisation est très avantageusement opérée par application de la méthode de linéarisations multiples que l'on va exposer ci-dessous, en référence à la figure 4.

A partir d'un point initial P(i) situé à une abscisse i (i = 7, les moyennes des tous premiers jours n'étant pas significatives), on considère un point P(j) à une abscisse j (j > i). Ce point définit sur la courbe C une corde P(i)P(j), soit un segment S1. On applique alors une formule de régression consistant à calculer, pour tous les points de la courbe C compris entre P(i) et P(j), la somme des valeurs absolues des écarts successifs e entre la courbe C et le segment S1. On augmente alors j, c'est-à-dire que l'on déplace vers la droite le point P(j), jusqu'à ce que la somme calculée à partir de la formule de régression atteigne une première valeur de seuil donnée (la première valeur de seuil, propre à chacune des trois courbes, est prédéterminée ou programmable et elle a été préalablement évaluée à partir de résultats d'essais cliniques).

Dès que l'on trouve une valeur j pour laquelle ce seuil est dépassé, on considère que le point P(j) correspondant constitue en principe la seconde extrémité du segment, celui-ci couvrant la période [i, j].

Toutefois, un critère additionnel est appliqué, en effectuant une vérification supplémentaire pour tous les points successifs compris entre P(i) et P(j) : si la distance entre le segment P(i)P(j) et la courbe C est, pour un point donné, supérieure à un second seuil prédéterminé, alors on remplace P(j) par ce nouveau point P(k). Le segment considéré devient alors le segment P(i)P(k), et non le segment P(i)P(j). En d'autres termes, si pour un point P(k) donné l'écart entre la courbe et le segment initial (résultant de l'application de la formule de régression) est supérieur à une limite donnée, on utilisera comme nouveau point celui correspondant à l'écart maximal.

En effet, le but de l'opération de segmentation est de faire apparaître des « dates remarquables » correspondant aux points d'articulation des segments successifs, et un lissage trop énergique de la courbe conduirait à éliminer de telles dates remarquables.

Ainsi, sur la figure 1, le segment S1 initialement considéré est remplacé par le segment S2, faisant apparaître la date remarquable k correspondant au point P(k).

L'algorithme est ensuite réitéré jusqu'à la fin de la courbe : le point P(k) sert de nouveau point de départ pour l'algorithme, qui recherche un segment S3 entre P(k) et un point P(I) susceptible de vérifier les critères indiqués ci-dessus, et ainsi de suite.

A la fin du processus, on obtient pour chacune des trois courbes W(i)/, VEact(i)/ et VErepos(i)/ une série de segments successifs, articulés entre eux autour de dates remarquables successives.

La figure 5 illustre la forme d'une telle caractéristique après segmentation.

L'étape suivante (bloc 24) consiste à calculer, pour chacune des trois courbes segmentées et pour chacune des périodes situées entre deux dates remarquables, un indice ci-après désigné « indice spécifique ».

Cet indice est avantageusement déterminé à partir des pentes des segments de droite successifs, par comparaison de chaque valeur de pente à deux valeurs limites, positive et négative, dites de « sensibilité de variation ». Si la pente est comprise entre ces deux valeurs, elle est considérée comme stable et l'indice '0' est attribué au segment correspondant ; dans le cas contraire on attribue un indice `+1' ou `-1', selon que la pente est positive ou négative.

La figure 5 illustre un exemple des variations de l'indice spécifique pour la série de segments de la courbe.

En d'autres termes, après avoir défini par segmentation un certain nombre de dates remarquables t1, t2, t3, t4 ..., on détermine un indice qui reflète la variation du paramètre considéré entre deux dates remarquables consécutives : peu ou pas de variation (indice spécifique = '0'), augmentation notable (indice spécifique = '+1') ou bien diminution notable (indice spécifique ='-1').

Ces données sont obtenues pour chacun des trois paramètres W, VEact et VErepos, mais avec des dates remarquables qui ne sont pas nécessairement les mêmes pour chacun d'entre eux.

L'étape suivante (bloc 26) consiste à déterminer un indice synthétique, ci-après désigné « indice composite », à partir des divers indices spécifiques et dates remarquables.

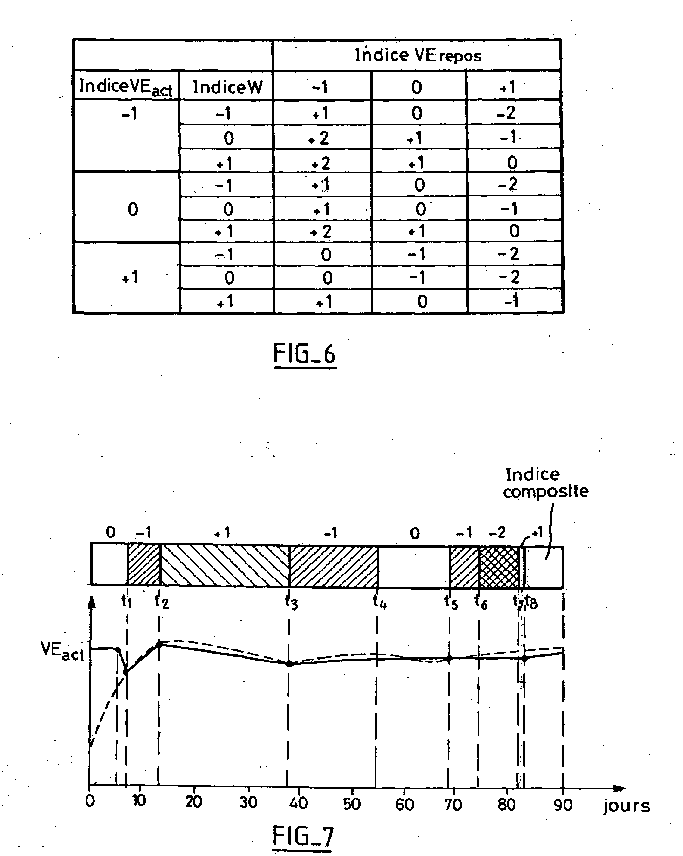

Cet indice composite est déterminé à partir d'une table de vérité combinant toutes les valeurs possibles des trois indices spécifiques précédemment déterminés. Cette table de vérité, dont une exemple est illustré figure 6, est établie en fonction d'avis médicaux et d'études cliniques et elle est censée refléter l'importance relative des divers paramètres lorsque ceux-ci sont combinés entre eux.

Cette table de vérité consiste à attribuer pour chaque jour une valeur '-2', `-1', '0', `+1' ou '+2' à l'indice composite. La valeur de ce dernier est donc susceptible de changer chaque fois qu'une date remarquable a été détectée pour l'un des trois paramètres W, VEact et VErepos.

Avantageusement, les changements de l'indice composite sont transposés sous forme graphique (bloc 28), à une étape qui peut être exécutée par le programmateur du praticien qui examine le patient, après que ce programmateur soit allé rechercher dans la mémoire du dispositif les données à afficher.

La figure 7 donne un exemple d'un tel affichage graphique, qui peut être présenté sous forme d'une barre colorée avec des couleurs différentes fonction de l'indice, par exemple : vert foncé > vert clair > blanc > jaune > rouge pour des valeurs respectives d'indice +2 > +1 > 0 > -1 > -2.

Ce code graphique est avantageusement affiché sur un écran en même temps que chacune, ou l'une, des trois courbes W(i)/, VEact(i)/ et VErepos(i)/, comme illustré sur la figure 7.

Ceci permet par exemple au praticien de repérer immédiatement une période de dégradation générale (en rouge), de déterminer les dates remarquables correspondant à cette évolution, et d'effectuer éventuellement un diagnostic plus précis par examen des variations de W, VEact et VErepos, et/ou des autres paramètres mémorisés par le dispositif au cours de la période considérée.

Par exemple, sur la figure 7, on voit que l'épisode d'aggravation compris entre t1 et t2 est très vraisemblablement lié au facteur VEact (t1 et t2 ne sont des dates remarquables pour VEact), tandis que l'épisode de forte aggravation compris entre t6 et t7 n'est pas lié à ce facteur (t6 et t7 ne sont pas des dates remarquables pour VEact), mais vraisemblablement à des variations sur les autres facteurs, non représentés sur cette figure.

Le praticien disposera ainsi immédiatement sur la période considérée de repères temporels précis qui pourront l'aider dans son diagnostic et dans l'interrogatoire de son patient.

## Revendications

1. Un dispositif médical implantable actif, notamment un dispositif de stimulation, resynchronisation, défibrillation et/ou cardioversion, ou un implant actif à visée diagnostique, comprenant :
- des moyens (10, 12) de mesure d'un paramètre corporel à prépondérance physiologique, notamment la ventilation-minute (VE), délivrant un signal physiologique,
- des moyens (14, 16) de mesure d'un paramètre corporel à prépondérance physique, notamment l'accélération (G), délivrant un signal physique,
- des moyens (18) discriminateurs des phases d'activité et de repos, opérant en réponse aux signaux physiologique et physique et délivrant un indicateur d'état du patient, et
- des moyens d'analyse (20-28), pour traiter et combiner les signaux physiologique et physique et l'indicateur d'état, et mémoriser les résultats obtenus sous forme d'un historique de données,
- des premiers moyens (20), pour établir des caractéristiques donnant, pour des dates successives, des valeurs représentatives sur une période donnée :
· du signal physique pendant les phases d'activité du patient,
· du signal physiologique pendant les phases d'activité du patient, et/ou
· du signal physiologique pendant les phases de repos du patient, dispositif
**caractérisé en ce que** les moyens d'analyse comprennent:
- des deuxièmes moyens (22), pour rechercher une pluralité de dates remarquables sur chacune des caractéristiques ainsi obtenues,
- des troisièmes moyens (24), pour attribuer des indices spécifiques d'état clinique respectifs à chacune des périodes comprises entre lesdites dates remarquables sur chacune des caractéristiques ainsi obtenues, et
- des quatrièmes moyens (26), pour combiner en un indice composite lesdits indices spécifiques ainsi déterminés.

2. Le dispositif de la revendication 1, dans lequel les deuxièmes moyens (22) comprennent des moyens de linéarisation multiple, pour transformer la caractéristique en une série continue de segments de droite, chaque extrémité de segment définissant l'une desdites dates remarquables pour cette caractéristique.

3. Le dispositif de la revendication 2, dans lequel les moyens de linéarisation multiple sont des moyens pour transformer la caractéristique en une série continue de segments de droite dont les extrémités sont des points de cette caractéristique.

4. Le dispositif de la revendication 2, dans lequel les moyens de linéarisation multiple sont des moyens itératifs opérant par application d'une formule de régression.

5. Le dispositif de la revendication 4, dans lequel les moyens de linéarisation multiple définissent une date remarquable si la somme des valeurs absolues des écarts entre segment et caractéristique dépasse un premier seuil prédéterminé.

6. Le dispositif de la revendication 5, dans lequel les moyens de linéarisation multiple ne définissent ladite date remarquable que si chacun des écarts entre segment et caractéristique n'excède pas un deuxième seuil prédéterminé.

7. Le dispositif de la revendication 2, dans lequel les troisièmes moyens (24) comprennent des moyens pour évaluer la pente desdits segments de droite et attribuer l'indice spécifique correspondant en fonction de la valeur de cette pente.

8. Le dispositif de la revendication 1, dans lequel les quatrièmes moyens (26, 28) sont des moyens mettant en oeuvre une table de vérité préenregistrée donnant de manière univoque, pour chaque combinaison possible d'indices spécifiques, une valeur correspondante d'indice composite.

9. Le dispositif de la revendication 1, dans lequel les premiers moyens (20) établissent lesdites caractéristiques à partir de moyennes mobiles des valeurs cumulées du signal physique pendant les phases d'activité, du signal physiologique pendant les phases d'activité, et/ou du signal physiologique pendant les phases de repos.

10. Le dispositif de la revendication 1, dans lequel lesdits indices spécifiques et composite d'état clinique sont réévalués à une périodicité quotidienne.

## Claims

1. An active implantable medical device, in particular a pacing, resynchronization, defibrillation and/or cardioversion device, or an active diagnosis implant, comprising:
- means (10, 12) for measuring a predominantly physiologic corporal parameter, in particular minute-ventilation (VE), which provide a physiologic signal;
- means (14, 16) for measuring a predominantly physical corporal parameter, in particular acceleration (G), which provide a physical signal;
- means (18) for discriminating rest and activity phases, operating in response to said physiologic and physical signals, which provide a patient status index; and
- analysis means (20-28), for processing and combining the physiologic and physical signals and the status index, and for memorizing the obtained results on the form of a data history;
- first means (20), for establishing characteristics providing, for successive dates, representative values over a given period of time, of:
the physical signal during the activity phases of the patient,
the physiologic signal during the activity phases of the patient and/or
.the physiologic signal during the rest phases of the patient,
said device being **characterized in that** the analysis means comprises:
- second means (22), for searching for a plurality of remarkable dates for each of the characteristics thus obtained,
- third means (24), for allocating specific indices of clinical status respective to each of the periods comprised between said remarkable dates for each of the characteristics thus obtained, and
- fourth means (26), for combining into one composite index, said specific indices thus determined.

2. The device of claim 1, wherein said second means (22) further comprises multiple linearization means, for transforming the characteristic into a continuous series of line segments, each segment endpoint defining one of said remarkable dates for said characteristic.

3. The device of claim 2, wherein said multiple linearization means are means for transforming the characteristic into a continuous series of line segments whose endpoints are points of this characteristic.

4. The device of claim 2, wherein said multiple linearization means are iterative means operating through the application of a regression formula.

5. The device of claim 4, wherein said multiple linearization means define a remarkable date if the sum of the absolute values of the differences between segment and characteristic is above a first predetermined threshold value.

6. The device of claim 5, wherein said multiple linearization means define said remarkable date only if each of the differences between segment and characteristic do not exceed a second predetermined threshold.

7. The device of claim 2, wherein said third means (24) comprise means for evaluating the slope of said line segments and for allocating the corresponding specific index as a function of the value of said slope.

8. The device of claim 1, wherein said fourth means (26, 28) further comprise means for implementing a pre-recorded truth table univalently providing, for each possible combination of specific indices, a corresponding value of composite index.

9. The device of claim 1, wherein said first means (20) establish said characteristics based upon moving averages of the accumulated values of the physical signal during the activity phases, of the physiologic signal during the activity phases, and/or the physiologic signal during the rest phases.

10. The device of claim 1, wherein said specific indices, and composite clinical status index, are re-evaluated with a given periodicity.

## Patentansprüche

1. Eine aktive implantierbare medizinische Vorrichtung, insbesondere eine Vorrichtung zur Stimulation, Resynchronisation, Defibrillation und/oder Kardioversion, oder ein aktives Implantat zur diagnostischen Absicht, welches umfasst:
- Messmittel (10, 12) eines körperlichen Parameters mit physiologischem Schwerpunkt, insbesondere Atmung pro Minute (VE), welche ein physiologisches Signal liefert,
- Messmittel (14, 16) eines Körperparameters mit physikalischem Schwerpunkt, insbesondere der Beschleunigung (G), welche ein physikalisches Signal liefert,
- Unterscheidungsmittel (18) der Aktivitäts- und Ruhephasen, welche in Antwort auf physiologische und physikalische Signale wirken und einen Indikator des Zustands des Patienten liefern, und
- Analysemittel (20 bis 28), um die physiologischen und physikalischen Signale und den Zustandsindikator zu verarbeiten und zu kombinieren, und um die erhaltenen Resultate in Form eines Verlaufs der Daten zu speichern,
- erste Mittel (20), um Merkmale festzulegen, welche für aufeinanderfolgende Zeitpunkte Werte geben, welche über eine gegebene Zeitspanne repräsentativ sind für:
das physikalische Signal während der aktiven Phasen des Patienten das physiologische Signal während der aktiven Phasen des Patientens und/oder Teile des physiologischen Signals während der Ruhezeiten des Patienten, wobei die Vorrichtung **dadurch gekennzeichnet ist, dass** die Analysermittel einschließen:
- zweite Mittel (22), um eine Vielzahl von bedeutenden Zeitpunkten der so erhaltenen Merkmale zu suchen,
- dritte Mittel (24), um spezifische Hinweise des klinischen Zustandes jedes entsprechenden Zeitraums, der zwischen den bedeutenden Zeitpunkten liegt, für jedes der so erhaltenen Merkmale zuzuordnen, und
- vierte Mittel (26), um die so bestimmten spezifischen Indexe in einem Kompositindex zusammenzufassen.

2. Die Vorrichtung nach Anspruch 1, in welcher die zweiten Mittel (22) Mittel zur Mehrfachlinearisierung einschließen, um das Merkmal in eine fortlaufende Serie von Rechtssegmenten umzuwandeln, wobei jeder Endpunkt des Segments einen der bedeutenden Zeitpunkte für dieses Merkmal definiert.

3. Die Vorrichtung aus Anspruch 2, in welcher die Mittel zur mehrfachen Linearisierung Mittel sind, um das Merkmal in eine fortlaufende Serie von Rechtssegmenten umzuwandeln, deren Endpunkte Punkte dieses Merkmals sind.

4. Die Vorrichtung nach Anspruch 2, in welcher die Mittel zur mehrfachen Linearisierung iterative Mittel sind, welche durch Anwendung einer Regressionsformel wirken.

5. Die Vorrichtung nach Anspruch 4, in welchem die Mittel mehrfacher Linearisisierung einen bedeutenden Zeitpunkt definieren, falls die Summe der absoluten Werte der Abstände zwischen Segment und Merkmal einen ersten vorbestimmten Grenzwert überschreitet.

6. Die Vorrichtung nach Anspruch 5, in welcher die Mittel zur mehrfachen Linearisierung nur die bedeutenden Zeitpunkte definieren, falls jeder der Abstände zwischen Segment und Merkmal nicht einen zweiten vorbestimmten Grenzwert überschreitet.

7. Die Vorrichtung nach Anspruch 2, in welchem die dritten Mittel (24) Mittel zum Berechnen der Steigung der Rechtssegmente und zum Zuweisen des entsprechenden spezifischen Index, in Abhängigkeit des Wertes dieser Steigung, mit einschließen.

8. Die Vorrichtung nach Anspruch 1, in welcher die vierten Mittel (26, 28) Mittel sind, welche eine vorgespeicherte Wahrheitstafel anwenden, welche in eindeutiger Weise, für jede mögliche Kombination spezifischer Indices, einen entsprechenden Kompositindexwert gibt.

9. Die Vorrichtung nach Anspruch 1, in welcher die ersten Mittel (20) die Merkmale, ausgehend von beweglichen Mittelwerten der kumulierten Werte des physikalischen Signals während der Aktivitätsphasen, des physiologischen Signals während der Aktivitätsphasen, und/oder des physiologischen Signals während der Ruhcphasen, festgesetzt werden.

10. Die Vorrichtung nach Anspruch 1, in welcher die spezifischen Indices und das Komposit des klinischen Zustands auf eine tägliche Periode neu bewertet werden.
